(19) Europäisches Patentamt
European Patent Office
Office européen des brevets

(11) Publication number: **0 462 662 A2**

# (12) EUROPEAN PATENT APPLICATION

(21) Application number: **91201489.1**

(51) Int. Cl.5: **C07J 73/00**, A61K 31/56

(22) Date of filing: **14.06.91**

A request for correction of the specification has been filed pursuant to Rule 88 EPC. A decision on the request will be taken during the proceedings before the Examining Division (Guidelines for Examination in the EPO, A-V, 2.2).

(30) Priority: **20.06.90 US 540968**

(43) Date of publication of application: **27.12.91 Bulletin 91/52**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Steinberg, Nathan G.**
**405 Willow Way**
**Clark, NJ 07066(US)**
Inventor: **Rasmusson, Gary H.**
**155 Park Place**
**Watchung, NJ 07060(US)**
Inventor: **Salzmann, Thomas N.**
**154 Meadowbrook Drive**
**North Plainfield, NJ 07062(US)**

(74) Representative: **Thompson, John Dr. et al**
**Merck & Co., Inc. European Patent Department Terlings Park Eastwick Road**
**Harlow, Essex CM20 2QR(GB)**

## (54) 17beta-N-monosubstituted adamantyl/norbornanyl carbamoyl-4-aza-5alpha-androst-1-en-3-ones and androstan-3-ones.

(57) 17β-N-Monosubstituted adamantyl/norbornanyl carbamoyl-4-aza-5α-androst-1-en-3-ones and androstan-3-ones of the formula:

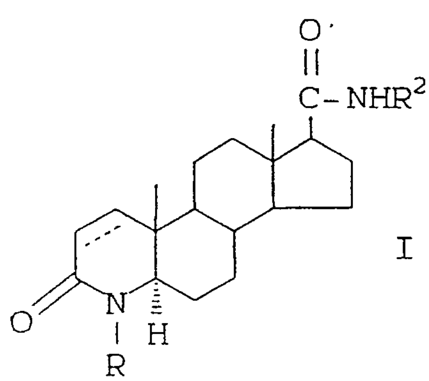

I

wherein R is selected from hydrogen, methyl and ethyl and $R_2$ is a hydrocarbon radical selected from substituted or unsubstituted 1- or 2-adamantyl or 1- or 2-norbornanyl and wherein the dotted line represents a double bond which can be present; are 5α-reductase inhibitors.

EP 0 462 662 A2

## BACKGROUND OF THE INVENTION

The present invention is concerned with novel 17β-N-monosubstituted adamantyl or norbornanyl carbamoyl-4-aza-5a-androst-1-en-3-ones and androstan-3-ones compounds and the use of such compounds as testosterone-5a-reductase inhibitors.

## DESCRIPTION OF THE PRIOR ART

It is well known in the art that certain undesirable physiological manifestations, such as acne vulgaris, seborrhea, female hirsutism, and male pattern baldness and benign prostatic hypertrophy, are the result of hyperandrogenic stimulation caused by an excessive accumulation of testosterone or similar androgenic hormones in the metabolic system. Early attempts to provide a chemotherapeutic agent to counter the undesirable results of hyperandrogenicity resulted in the discovery of several steroidal antiandrogens having undesirable hormonal activities of their own. The estrogens, for example, not only counteract the effect of the androgens but have a feminizing effect as well. Non-steroidal antiandrogens have also been developed, for example, 4'-nitro-3'-trifluoromethylisobutyranilide. See Neri et al., Endo., Vol. 91, No. 2 (1972). However, these products, though devoid of hormonal effects, are peripherally active, competing with the natural androgens for receptor sites, and hence have a tendency to feminize a male host or the male fetus of a female host.

It more recently became known in the art that the principal mediator of androgenic activity in some target organs is 5a-dihydrotestosterone, and that it is formed locally in the target organ by the action of testosterone-5a-reductase. It therefore has been postulated and demonstrated that inhibitors of testosterone-5a-reductase will serve to prevent or lessen symptoms of hyperandrogenic stimulation. Nayfe et al., Steroids, 14, 269 (1969) demonstrated in vitro that methyl 4-androsten-3-one-17β-carboxylate was a testosterone-5a-reductase inhibitor. Then Voigt and Hsia, Endocrinology, 92, 1216 (1973), Canadian Pat. No. 970,692, demonstrated that the above ester and the parent free acid, 4-androsten-3-one-17β-carboxylic acid are both active inhibitors of testosterone-5a-reductase in vitro. They further demonstrated that topical application of either testosterone or 5a-dihydrotesterone caused enlargement of the female hamster flank organ, an androgen dependent sebaceous structure. However, concommitant administration of 4-androsten-3-one-17β-carboxylicacid or its methyl ester inhibited the response elicited by testosterone but did not inhibit the response elicited by 5a-dihydrotestosterone. These results were interpreted as indicating that the compounds were antiandrogenic by virtue of their ability to inhibit testosterone-5a-reductase.

A number of 4-aza steroid compounds are known. See, for example, U.S. Pat. Nos. 2,227,876; 3,239,417; 3,264,301; and 3,285,918; French Pat. No. 1,465,544; Doorenbos and Solomons, J. Pharm. Sci. 62, 4, pp. 638-640 (1973); Doorenbos and Brown, J. Pharm. Sci., 60 8, pp. 1234-1235 (1971); and Doorenbos and Kim, J. Pharm. Sci. 63, 4, pp. 620-622 (1974).

In addition, U.S. Patents 4,377,584, 4,220,775, 4,859,681, 4,760,071 and the articles J. Med. Chem. 27, p. 1690-1701 (1984) and J. Med. Chem. 29, 2998-2315 (1986) of Rasmusson et al., U.S. Patent 4,845,104 to Carlin et al. and U.S. Patent 4,732,897 to Cainelli et al. describe 4-aza-17β-substituted-5a-androstan-3-ones which are said to be useful in the treatment of hyperandrogenic conditions. However, none of the cited references suggest that any of the novel 17βN-(monosubstituted) adamantyl carbamoyl-4-aza-5a-androsten-1-en-3-ones or androstan-3-ones of the present invention would have utility as highly potent testosterone-5a-reductase inhibitors.

## SUMMARY OF THE INVENTION

The present invention relates to novel 17β-N-(monosubstituted) adamantyl and norbornanyl carbamoyl-4-aza-5a-androsten-1-en-3-ones and androstan-3-ones compounds, processes for their preparation, pharmaceutical formulations comprising these novel compounds as active ingredients and methods of inhibiting testosterone-5a-reductase and of treating hyperandrogenic conditions with the novel compounds and their pharmaceutical formulations.

In accordance with this invention, there is provided compounds of the formula:

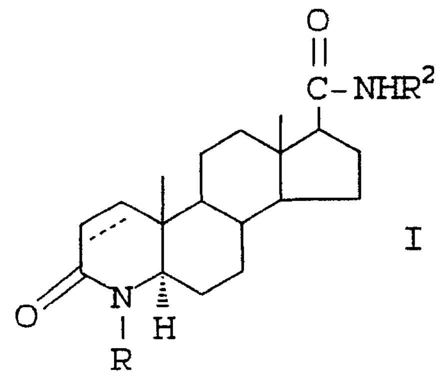

I

wherein

R is hydrogen, methyl or ethyl;

$R^2$ is a hydrocarbon radical selected from substituted or unsubstituted 1- or 2-adamantyl or 1-or 2-norbornanyl, the dotted line represents a double bond which can be present, and pharmaceutically acceptable salts or esters thereof.

## BRIEF DESCRIPTION OF THE INVENTION AND PREFERRED EMBODIMENTS

Representative compounds of the present invention include the following:

17β-(N-2-adamantyl-carbamoyl)-4-aza-5-a-androst-1-en-3-ones;
17β-(N-2-adamantyl-carbamoyl)-4-aza-4-methyl-5-a-androst-1-en-3-ones;
17β-(N-1-adamantyl-carbamoyl)-4-aza-5a-androst-an-3-ones;
17β-(N-1-adamantyl-carbamoyl)-4-aza-4-methyl-5a-androst-1-en-3-ones;
17β-(N-1-adamantyl-carbamoyl)-4-aza-4-methyl-5-a-androst-an-3-ones;
17β-(N-2-norbornanyl-carbamoyl)-4-aza-4-methyl-5a-androst-1-en-3-ones;
17β-(N-2-norbornanyl-carbamoyl)-4-aza-5a-androst-1-en-3-ones; and
the corresponding compounds wherein the substituent is replaced in each of the above named compounds by a hydrogen, methyl or an ethyl radical, to form a different N-substituent, and wherein a double bond can be optionally present as indicated by the dotted line in position 1.

The 1-, 2-adamantyl or 1-, 2-norbornanyl moieties can be substituted with one or more substituents of the following: $C_1$-$C_4$ linear/branched alkyl, including methyl, ethyl, isopropyl, n-butyl; nitro; oxo; $C_7$-$C_9$ aralkyl, including benzyl; $(CH_2)n$ COOR where n is 0-2 and R is H or $C_1$-$C_4$ linear/branched alkyl including methyl, ethyl; $CH_2OH$; OH; OR where R is $C_1$-$C_4$ linear/ branched
alkyl including methyl, ethyl; halo, including fluoro, bromo, iodo; COOH; COOR, where R is linear/branched $C_1$-$C_4$ alkyl; $-CONH_2$; $CH_2NH_2$; $CH_2NHCOR$ where R is $C_1$-$C_4$ linear/branched alkyl including methyl, ethyl; phenyl; o, m, p-substituted phenyl including p-nitro, p-amino and p-sulfo; or cyano.

Also included within the scope of this invention are pharmaceutically acceptable salts or esters, where a basic or acidic group is present on the adamantyl or norbornanyl moiety. When an acidic substituent is present, i.e. -COOH, there can be formed the ammonium, sodium, potassium, calcium salt, and the like, for use as the dosage form.

Where a basic group is present, i.e. amino, acidic salts, i.e. hydrochloride, hydrobromide, acetate, pamoate, and the like, can be used as the dosage form.

Also, in the case of the -COOH group being present, pharmaceutically acceptable esters can be employed, e.g. acetate, maleate, pivaloyloxymethyl, and the like, and those esters known in the art for modifying solubility or hydrolysis characteristics for use as sustained release or prodrug formulations.

Representative examples include for $R^2$ (where AD is adamantyl):
3, 5, 7-trinitro-1-AD; 4-oxo-1-AD; 1-benzyl-1-AD; 4,4-dimethyl-1-Ad; 3,7-dimethyl-5-carboxymethyl-1-AD; 3-carboxymethyl-1-AD; 3-chloro-1-AD; 1,3-dihydroxy-6,6-dimethyl-2-AD; 3-chloro-1-AD; 4-carbethoxy-2-AD; 4-carboxy-2-AD; 3-isopropyl-1-AD; 3-n-butyl-1-AD; 3-propyl-1-AD; 3-,5-diethyl-1-AD; 3-hydroxymethyl-1-AD; 2-carboxy-1-AD; 3-methyl-1-AD; 5-hydroxy-2-AD; 2-hydroxy-1-AD; 1-aminomethyl-1-hydroxy-2-AD; 2-oxo-1-AD; 2-phenyl-2-AD; 1-amino-methyl-2-AD; 1-carboxy-2-AD; 1-aminocarbonyl-2-AD; 3-hydroxy-5,7-dimethyl-1-AD; 4-fluoro-1-AD; 3-fluoro-1-AD; 4-hydroxy-2-AD; 3-phenyl-1-AD; 3-(p-aminophenyl)-1-AD; 3-(p-nitrophenyl)-1-AD; 3-methyl-5-hydroxymethyl-1-AD; 3,5-dimethyl-4-hydroxy-1-AD; 2-hydroxymethyl-2-AD; 3-(p-sulfophenyl)-1-AD; 3-methyl-5-ethyl-1-AD; 2-carboxy-2-AD; 3,5-7-trimethyl-1-AD; 4-iodo-2-AD; 4-bromo-2-AD; 4-chloro-2-AD; 1-acetylaminomethyl-2-AD; 1-carboxymethyl-2-AD; 1-methyl-2-AD; 1-aminocarboxylmethyl-2-AD; 1-aminocarboxyl-1-AD; 2-cyano-2-AD; 3,5-dimethyl-7-ethyl-1-AD; 4-hydroxy-1-AD; 1-hydroxy-2-AD; 5-carboxy-3-methyl-1-AD; 3,5-dimethyl-7-carboxy-1-AD; 3-carboxy-1-AD; 3-hydroxy-1-AD; and the like.

Representative examples include for $R^2$ as substituted norbornanyl moieties are (where NB is norbornanyl):

2-NB; 1,7,7-trimethyl-4-phenyl-2-NB; 3-carboxy-2-NB; 3-phenyl-2-carboxy-2-NB; 2-cyano-3-phenyl-2-NB; 3-hydroxy-4,7,7-trimethyl-2-NB; 6-hydroxymethyl-2-NB; 5-cyano-2-NB; 3-allyl-2-NB; 1-NB; 7,7-dimethyl-1-hydroxymethyl-2-NB; 3-methoxy-4,7,7-trimethyl-2-NB; 3-aminocarbonyl-2-NB; 3-ethoxycarbonyl-2-NB; 3,3-dimethyl-2-NB; 7-oxo-1-NB; 3-phenyl-2-NB; 1-carboxy-methyl-7,7-dimethyl-2-NB; 1-ethyl-2-NB; 1-methyl-2-NB; 2,2,3,3,5,5,6,6,7,7-decafluoro-1-NB; 3-hydroxy-2-NB; 3-chloro-2-NB; 3-(p-methoxyphenyl)-2-NB; 2,2-dimethyl-3-methylene-7-NB; 3-oxo-2-NB; 1-methoxy-2-NB; 7-NB; 3-isopropyl-2-NB; 2-bromo-1-NB; 3-chloro-1-NB; and the like.

Procedures for preparing the adamantyl and norbornanyl amines useful in this invention, including the above, are well known in the art.

The novel compounds of formula I of the present invention can be prepared by a method starting with the known steroid ester (II) of the formula:

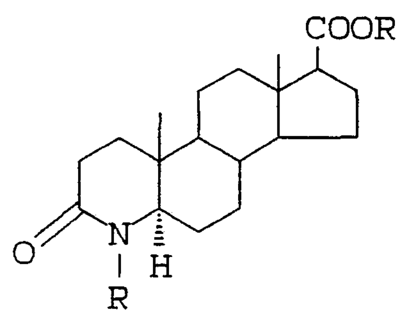

II

17β-(carbomethoxy)-4-aza-5-a-androstan-3-ones which includes the stages of optionally 1) dehydrogenating said starting material to produce the corresponding compound containing a double-bond in the 1,2-position of the A-ring, 2) converting the 17-carbomethoxy substituent into an N-monosubstituted adamantyl-carbamoyl substituent and, if desired, 3) alkylating the A-ring nitrogen to introduce a N-methyl or N-ethyl substituent into the A ring 4-position. For the dehydrogenatin step, it is preferable that the 4-aza nitrogen be unsubstituted. The alternate pathways can consist of one or more discrete chemical steps and if desired can take place before step (1) or following step (1) or step (3).

In accordance with the process of the present invention (see flow sheet), the products of our invention are formed by optionally, 1) heating a 17β-alkoxycarbonyl-4-aza-5a-androstan-3-ones, compound III, (prepared in the literature as described in the reference US Patent 4,377,584) with a dehydrogenating agent such as benzeneseleninic anhydride in a refluxing inert solvent, e.g. chlorobenzene, to form a 17β-alkoxycarbonyl-4-aza-5a-androst-1-ene-3-one IV (alternately, the dichlorodicyanobenzoquinone process of Dolling, et al., JACS 1988, Vol. 110, pp. 3318-3319, can be used), 2) the formed 5a-androst-1-en-3-one compound from Step 1 can be reacted with, e.g. sodium hydride under anhydrous conditions in a neutral solvent such as dimethylformamide, 3) contacting the resulting reaction mixture with an alkyl (methyl or ethyl) iodide to form the corresponding 17-β-alkoxy-adamantyl-carbamoyl-4-alkyl-4-aza-5a-androst-1-en-3-one V, 4) subsequently hydrolyzing said 17β-alkoxycarbonyl-4-alkyl-4-aza-5a-androst-1-en-3-one with a strong base, such as aqueous methanolic potassium hydroxide at the reflux temperature, followed by acidification and isolation of the resulting steroidal acid to yield 17β-carboxy 4-alkyl-4-aza-5a-androst-1-en-3-one VI, 5) said steroidal acid can be then converted to its corresponding 2-pyridylthio ester by refluxing with triphenyl phosphine and 2,2'-dipyridyl disulfide in an inert solvent such as toluene and the resulting product 17β-(2-pyridylthiocarbonyl)-4-alkyl-4-aza-5a-androst-1 -en-3-one VII can be isolated by chromatography on e.g. silica gel, 6) said pyridylthio ester can be then reacted with 1-adamantyl-, 2-adamantylamine or norbornanylamine in an inert solvent e.g. tetrahydrofuran, to form the desired product 17β-N-adamantyl-carbamoyl-4-alkyl-4-aza-5a-androst-1-en-3-one VIII which can be isolated by chromatography e.g. on silica gel. When the previous reaction is carried out in the absence of first forming the double bond at position 1, the corresponding 17β-(N-adamantylcarbamoyl)-4-alkyl-4-aza-5a-androstan-3-one (or N-norbornanyl carbamoyl compound) is prepared.

In accordance with an alternate process of our invention the corresponding N-unsubstituted-17$\beta$(N-adamantyl-carbamoyl)-4-aza-5a-androst-1-en-3-one XIV is readily prepared from the 17$\beta$ (alkoxycarbonyl)-4-aza-5a-androstone-3-one IV by repeating the above series of reaction steps but omitting the alkylation Step 2 herein above, i.e. treatment of the 4-aza-5-a-androst-1-en-3-one with e.g. sodium amide followed by methyl or ethyl iodide via intermediates XII and XIII.

In accordance with a further alternate process of preparing the compounds of our invention having only hydrogen as the sole substituent on the ring A - nitrogen, the double bond in the A ring is introduced as the last step of the process. Thus, a 17$\beta$-alkoxycarbonyl 4-aza-5a-androstan-3-one III is hydrolyzed to the corresponding steroidal acid IX 17$\beta$-carboxy-4-aza-5a-androstan-3-one which in turn is converted to the corresponding pyridylthio ester, 17$\beta$ (2-pyridylthiocarbonyl)-4-aza-5a-androstan-3-one, X followed by treatment of the ester with an amine of formula $R^2$-$NH_2$ wherein $R^2$ is as defined hereinabove as 1- or 2-adamantyl or 1-, 2-, or 7-norbornanyl to form a 17$\beta$ (N-adamantyl-carbamoyl)-4-aza-5a-androstone-3-one XI which is dehydrogenated as previously described to produce compound XIV, 17$\beta$-(N-adamantyl-carbamoyl)-4-aza-androst-1-en-3-one or corresponding norbornanyl derivative.

In another alternate method of introducing the 17$\beta$-(N-adamantyl-carbamoyl)substituent into a 17$\beta$-carboxy androstane compound of formula VI, XII or IX, each is treated in a manner similar to the procedure described in Steroids, Vol. 35 #3, March 1980, p. 1-7 with dicyclohexylcarbodiimide and 1-hydroxybenzotriazole to form the 17$\beta$-(1-benzotriazoloxycarbonyl)-4-aza-5a-androst-1-en-3-one, VII, XIII or X, wherein X is 1-benzotriazoloxy or 17$\beta$-(1-benzotriazoloxy-carbonyl)-4-aza-5a-androstan-3-one, X.

The above reactions are schematically represented in the following flowsheet.

## Flowsheet

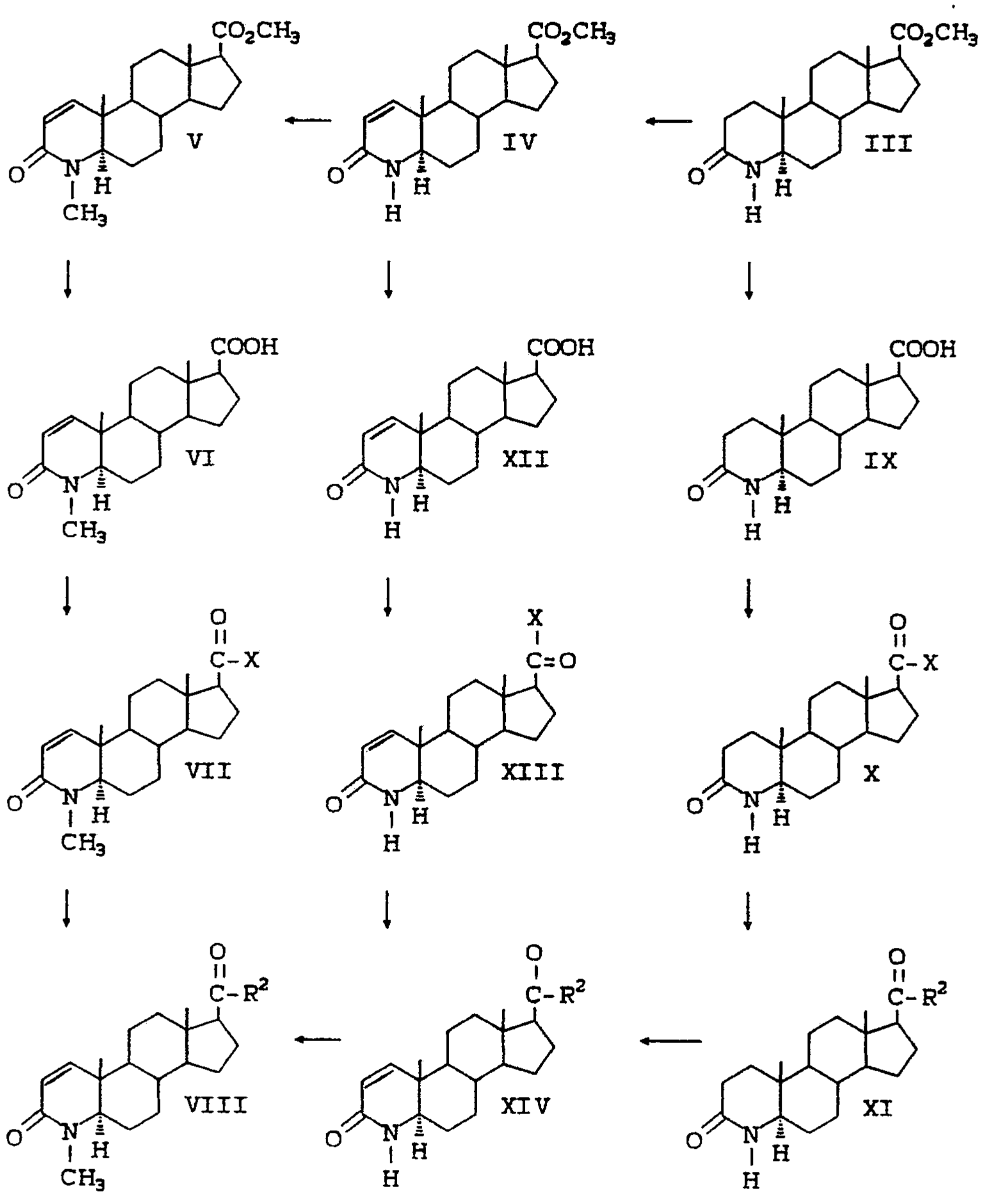

V IV III

VI XII IX

VII XIII X

VIII XIV XI

X is 2-pyridylthio or 1-benzotriazoloxy.

$R^2$ is 1- or 2-adamantyl or norbornanyl.

The compounds of the present invention, prepared in accordance with the method described above, are, as already described, potent antiandrogens by virtue of their ability to specifically inhibit testosterone-5a-reductase.

Accordingly, the present invention is particularly concerned with providing a method of treating the hyperandrogenic conditions of acne vulgaris, seborrhea, and female hirsutism by topical administration, and a method of treating all of the above conditions as well as benign prostatic hypertrophy, by oral or parenteral administration, of the novel compounds of the present invention.

The present invention is thus also concerned with providing suitable topical, oral and parenteral pharmaceutical formulations for use in the novel methods of treatment of the present invention.

The compositions containing the compounds of the present invention as the active ingredient for use in the treatment of benign prostatic hypertrophy can be administered in a wide variety of therapeutic dosage

forms in conventional vehicles for systemic administration, as, for example, by oral administration in the form of tablets, capsules, solutions, or suspensions, of by intravenous injection. The daily dosage of the products may be varied over a wide range varying from 50 to 2,000 mg. The compositions are preferably provided in the form of scored tablets containing 5, 10, 25, 50, 100, 150, 250, and 500 milligrams of the active ingredient for the symptomatic adjustment of the dosage to the patient to be treated. An effective amount of the drug is ordinarily supplied at a dosage level of from about 1 mg. to about 50 mg./kg. of body weight per day. Preferably the range is from about 1 mg. to 7 mg./kgs. of body weight per day. These dosages are well below the toxic dose of the product. Capsules containing the product of this invention can be prepared by mixing an active compound of the present invention with lactose and magnesium stearate, calcium stearate, starch, talc, or other carriers, and placing the mixture in gelatin capsule. Tablets may be prepared by mixing the active ingredient with conventional tableting ingredients such as calcium phosphate, lactose, corn starch or magnesium stearate. The liquid forms in suitably flavored suspending or dispersing agents such as the synthetic and natural gums, for example, tragacanth, acacia, methyl-cellulose and the like. Other dispersing agents which may be employed include glycerin and the like. For parenteral administration, sterile suspensions and solutions are desired. Isotonic preparations which generally contain suitable preservative are employed when intravenous administration is desired.

For the treatment of acne vulgaris, seborrhea, female hirsutism, the compounds of the present invention are administered in the formula of pharmaceutical composition comprising the active compound in combination with a pharmacologically acceptable carrier adapted for topical administration. These topical pharmaceutical compositions may be in the form of a cream, ointment, gel or aerosol formulation adapted for application to the skin. These topical pharmaceutical compositions containing the compounds of the present invention ordinarily include about 0.1% to 15%, preferably about 5%, of the active compound, in admixture with about 95% of vehicle.

The method of preparing the novel 17β-N-monosubstituted adamantyl-carbamoyl compounds of the present invention, already described above in general terms, may be further illustrated by the following examples.

EXAMPLE 1

Methyl 3-oxo-4-aza-5a-androst-1-ene-17β-carboxylate

A suspension of 83.7 g of methyl 3-oxo-4-aza-5a-androstane-17-carboxylate (III) (Rasmusson, Johnston and Arth. U.S. Patent 4,377,584, March 22, 1983.) and 126.5 g of benzeneseleninic anhydride in 2.09 1 of chlorobenzene was heated at reflux for 2 hours. The reflux condenser was switched to a distillation head and the mixture was distilled slowly to remove water that had formed in the reaction (2 hours). The solution was evaporated to leave 198 g of wet residue. The residue as a solution in dichloromethane was washed with saturated aqueous $NaHCO_3$ solution and saturated NaCl solution, then dried and evaporated to leave 172.4 g. This material was chromatographed on 2.56 kg of silica gel eluting first with dichloromethane (5 liters) and then with 4:1 dichloromethane acetone. The desired product was eluted with 8 liters of the above-mixed solvent and evaporated in vacuo to yield 53.4 g. It was washed with diethyl ether and dried to leave 49.5 g of the unsaturated title product, m.p. 278-280° C.

EXAMPLE 2

Synthesis of 17β(N-2-adamantyl-carbamoyl)-4-aza-5a-androst-1-en-3-one

200.0 mg (0.609 mmoles) of the unsaturated ester product from Example 1 was suspended in 30.0 ml of dry THF, and to the suspension was added 366.0 mg (2.4 mmoles) of 2-adamantanamine under $N_2$ atmosphere. The reaction was cooled to 0-5° C and 1.18 ml of 1.0M solution of EtMgBr in THF was added under $N_2$. The resulting mixture was allowed to stir for 10 minutes and then refluxed for 2-1/2 hours/$N_2$. The reaction mixture was cooled to 0° C, and quenched with a saturated solution of $NH_4Cl$. The organic layer was separated, and the aqueous layer was extracted 3x with $CH_2Cl_2$. The organic layers were combined, washed 2 times with water, 2 times with saturated NaCl solution, dried over $MgSO_4$, filtered, and evaporated under vacuum to yield crude product. Crystallization from EtOAc afforded pure above-titled product, 146.0 mg, m.pt 305-305° C. The FAB (Fast atom bombardment) gave a molecular weight $M^+ + 1 = 451$; Calc $M^+ + 1 = 451$.

Anal. Calcd for $C_{29}H_{42}N_2O_2$:
C,77.28; H,9.40; N,6.21
Found: C,76.84; H,9.73; N,5.93.

EXAMPLE 3

Synthesis of 17β(N-1-adamantyl-carbamoyl)-4-aza-5a-androst-1-en-3-one

100 mg of the 17-methyl ester (0.305 mmoles) from Example 1 was suspended in 3.0 ml of THF (dried over molecular sieves 3A), and then was added 183.0 mg of 1-adamantanamine (1.2 mmoles). The suspension was cooled to 5-10 °C and then 590 ml of 2.0 M solution, of EtMgBr in THF was added. The resulting mixture was allowed to stir for 10 minutes, and then refluxed for 1-2 hours under $N_2$. The mixture was cooled to 0 °C and then quenched with saturated solution of $NH_4Cl$ (about 10 ml.). The organic layer was separated and the aqueous layer extracted with three volumes $CH_2Cl_2$.

The organic layers were combined, washed 2 times with $H_2O$, twice with saturated sodium chloride, and dried over $MgSO_4$, filtered and evaporated to dryness in vacuum. Crystallized from EtOAc afforded 75.0 mg of product, mpt. 305-306 °C. Molecular weight (by FAB) showed $M^+$ =451: Found =451.

Anal. Calcd. for $C_{29}H_{42}N_2O_2$:
C,77.28; H,9.40; N,6.21.
Found: C,76.84; H,9.73; N,5.93.

EXAMPLE 4

Synthesis of 17β(N-1-Adamantyl Carbamoyl)-4-aza-5a-androstane-3-one

100.0 mg of the adamantyl derivative produced in Example 3 was dissolved in 5.0 ml of dry THF. 300 mg of 5% Pd/C was added and hydrogenated for 6.0 hrs. at R.T. at 40 psi. The mixture was filtered through celite, the cake washed with THF (3 times) and solvent evaporated under vacuum to yield 97.0 mg. of crude above-titled product. NMR showed absence of olefins. The crude material was placed on 15.0 g silica gel column, and eluated with 1:1($CH_2Cl_2$: acetone).

Collected fractions afforded a single spot material by TLC weighing 77.98 mg. NMR was in excellent agreement with the proposed structure. Recrystallized from EtOAc to yield 65.59 mg of the above-titled product, mp. 323-324 °C.

Anal. Calcd. for $C_{29}H_{44}O_2N_2$ 1/4 $H_2O$:
C,76:81; H,9.81; N,6.13.
Found: C,75.91; H,9.97; N,6.06.

EXAMPLE 5

Methyl 3-oxo-4-methyl-4-aza-5a-androst-1-ene-17β-carboxylate

A suspension of 25 g of the product of Example 1 and 2.25 g of sodium hydride in 500 ml of dry dimethylformamide was stirred under nitrogen for 15 minutes. Methyl iodide (15 ml) was added dropwise and the mixture was stirred for 30 minutes at room temperature. Additional (5 ml) methyl iodide was added and the mixture was heated at 50 °C for 2 hours. After cooling the mixture was diluted with water to 2 liters. The solid was separated after cooling and amounted to 25.4 g of the above-titled product, m.p. 159-161 °C.

EXAMPLE 6

S-(2-Pyridyl)-3-oxo-4-methyl-4-aza-5a-androst-1-ene-17β-thiocarboxylate

6(A) A suspension of 25 g of the product of Example 5 in 125 ml of methanol was treated with a solution of KOH (12.5 g) in 12.5 ml of water. After refluxing for 4 hours, the solution was acidified with 6 NHCl and then was diluted with water. The crude acid (23.32 g) was separated, dried and had a m.p. 300° C.

6(B) The crude, dry acid (23 g), triphenylphosphine (36.45 g) and 2,2'-dipyridyldisulfide (30.4 g) were suspended in 138 ml of toluene with stirring overnight at room temperature. Next day, crystallization set in. The reaction mixture was filtered, washed with cold toluene, followed with cold anhydrous ether. Dried at 110° C in vacuo to afford 20.4 g of the desired above-titled thiopyridyl ester m.pt. 218-220° C.

EXAMPLE 7

Synthesis of 17β(N-1-adamantyl-carbamoyl)-4-methyl-4-aza-5a-androst-1-en-3-one

120 mg of the thiopyridyl ester of Example 6 was suspended in 20 ml of dry THF, to the suspension was added 175.0 mg of 1-adamantanamine under $N_2$. The reaction was carried out at R.T. for 16 hours under $N_2$. The reaction was monitored by TLC, using 1:1 acetone: hexane. After 6 hrs. the TLC showed that the reaction went exclusively to the product, with trace of starting material left behind. The product was separated on TLC 20 cm x 20 cm, 1000 mm silica gel plate, eluated with 1:1 (acetone/hexane). The product was crystallized from ethyl acetate, to give 50.0 mg of pure material m. pt. 202-205° C. Molecular Weight (FAB) showed 465; Calc: 465. Recrystallization afforded 19.14 mg of the above-titled product, m.pt. 202-202.5° C.

Anal. Calcd for $C_{30}H_{44}N_2O_2H_2O$:
C,74.64; H,9.60; N,5.80.
Found: C,74.32; H,9.47; N,5.89.

EXAMPLE 8

Hydrolysis of Methyl-3-oxo-4-aza-5a-androstane-17β-carboxylate

The 17β-androstane carboxylate starting material of Example 1 was hydrolyzed with 7% aqueous KOH in isopropanol or aqueous methanol, followed by an acidic work-up to give the corresponding 17β carboxylic acid which was utilized in Example 9.

EXAMPLE 9

N-(1-adamantyl)-3-oxo-4-aza-5a-androstane-17β-carboxamide

A solution of 5.0 g of the product of Example 8, 3.35 g of dicyclohexylcarbodiimide and 3.18 g of 1-hydroxybenzotriazole in 500 ml of dichloromethane was stirred at room temperature overnight. The solid was separated by filtration and the filtrate was treated with 1-adamantamine. This solution stood at room temperature for 64 hours, then filtered, and the solution was washed successively with 10% hydrochloric acid and saturated aqueous sodium chloride. After drying with $MgSO_4$, it was filtered and concentrated. The crude product was eluted through 240 g of silica gel with 3:7 (acetone-dichloromethane) to give 5.5 g of the above-titled product, m.p. 250-251° C.

EXAMPLE 10

Synthesis of 17β(N-1-adamantyl-carbamoyl)-4-methyl-4-aza-5a-androstan-3-one

A suspension of 83.7 g of methyl-3-oxo-4-methyl-4-aza-5a-androstane-17β-carboxylate III (Rasmusson,

Johnston and Arth U.S. Patent 4,377,584, March 22, 1983) was hydrolyzed with 7% aqueous KOH in isopropanol or aqueous methanol, followed by an acidic work up to give the corresponding 17β-carboxylic acid.

The acid was readily converted into benzotriazoyl-1-yl-3-oxo-4 methyl-4-aza-5a-androstane 17β carboxylate as described in Example 9. The activated ester (the benzotriazoyl derivative) was purified on TLC (4 plates, 20 cm x 20 cm x 20 cm x 1000mm silica gel) eluted with 4:96 (MeOH-$CHCl_3$). The isolated product was washed with ether to give 192.0 mg of the active ester m.pt. 217-219°C with decomposition.

EXAMPLE 11

Synthesis of 17β (N-1 adamantyl-carbamoyl)-4-methyl-4-aza-5a-androstan-3-one

100.0 mg of the 4-methyl-4-aza-benzotriazole derivative prepared as described in Example 10, was dissolved in 20.0 ml $CH_2Cl_2$. To the clear solution was added 127 mg of 1-adamantamine. The reaction mixture was stirred overnight at R.T./$N_2$.

Crystallization from EtOAc after filtering the solution through Teflon Acrodisc CR afforded 26-32 mg, m.pt. 210-217°C. The product was further purified on 1.0 g silica gel column (EM silica gel) with 1:1 (acetone-hexane) as eluant to give after recrystallization 21.75 mg of white needles of the above-titled product, m.pt. 203-205°C.

Anal. Calcd. for $C_{30}H_{46}N_2O_2$ " 1.5 $H_2O$:
C,73.58; H,9.68; N,5.62;
Found: C,73.15; H,9.30; N,5.67.

EXAMPLE 12

Diastereomeric Synthesis of 17β(N-exo-2-norbornanyl-carbamoyl)-4-aza-5a-androst-1-en-3-one)

100.0 mg of the thiopyridyl ester of Example 6 was dissolved in 3.0 ml of dry THF under $N_2$. To the clear solution was added 477 ml of (±) racemic exo-2-aminonorbornane. Allowed the reaction to proceed for 16 hours at R.T./$N_2$. The reaction mixture was evaporated to dryness in vacuum. The residue was dissolved in chloroform. The organic layer was washed with 2.5 N HCl acid (3 times); 3 times with water; 3 times with saturated NaCl solution, dried over $MgSO_4$, filtered and evaporated to dryness in vacuum to afford 56.3 mg of a diastereomeric mixture.

The crude product was chromatographed on TLC (2 plates, 20 cm x 20 cm x 500 mm silica gel) eluted with 70:30 ($CHCl_3$:acetone) to yield 70.0 mg.of the above-titled product.

NMR ($CDCl_3$) confirmed the above structure.

FAB calcd. for $C_{26}H_{38}O_2N_2$: 411; Found: 411.

Anal. Calcd. for $C_{26}H_{38}O_2N_2$ " 1 mole of $H_2O$:
C,72.82; H,9.40; N,6.58.
Found: C,73.21; H,9.20; N,6.25.

**Claims**

1. A compound of the formula:

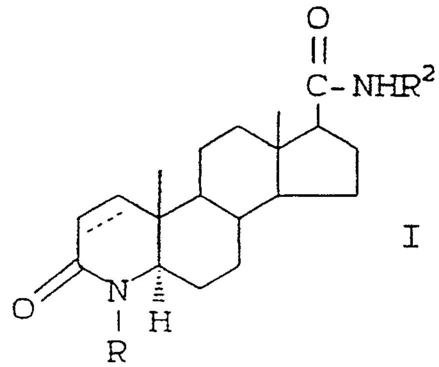

I

wherein R is hydrogen, methyl or ethyl; $R^2$ is a hydrocarbon radical selected from substituted or unsubstituted 1- or 2-adamantyl, 1- or 2-norbornanyl, wherein the dotted line represents a double bond which can be present, and pharmaceutically acceptable salts or esters thereof.

**2.** The compound of Claim 1 wherein the 1-or 2-adamantyl or 1- or 2-norbornanyl moieties are substituted with one or more of: $C_1$-$C_4$ linear or branched alkyl, nitro, oxo, $C_7$-$C_9$ aralkyl, $(CH_2)n$ COOR where n is 0-2 and R is H or linear/branched $C_1$-$C_4$ alkyl, $CH_2OH$, OH, OR where R is $C_1$-$C_4$ linear/branched alkyl, halo, COOH, COOR where R is linear/branched $C_1$-$C_4$ alkyl, $CONH_2$, $CH_2NH_2$, $CH_2NHCOR$ where R is $C_1$-$C_4$ linear/branched alkyl, phenyl, p-nitrophenyl, p-aminophenyl, p-sulfophenyl or cyano.

**3.** A pharmaceutical composition comprising a pharmaceutically acceptable carrier and a therapeutically effective amount of a compound according to Claim 1.